# EUROPÄISCHE PATENTSCHRIFT

(11) **EP 2 421 818 B1**
(45) Veröffentlichungstag und Bekanntmachung des Hinweises auf die Patenterteilung: **27.08.2014**
(21) Anmeldenummer: 10713992.5
(22) Anmeldetag: 20.04.2010
(51) Int. Cl.: C07C 209/78, C07C 263/10, C07C 211/50, C07C 265/14

(54) **VERFAHREN ZUR HERSTELLUNG VON FARBSTABILEM MDA UND MDI**
METHOD FOR PRODUCING COLOR-STABLE MDA AND MDI
PROCÉDÉ DE PRODUCTION DE MDA ET DE MDI AUX COULEURS STABLES

(30) Priorität: 24.04.2009 EP 09158741
(43) Veröffentlichungstag der Anmeldung: 29.02.2012
(73) Patentinhaber: BASF SE, 67056 Ludwigshafen (DE)
(72) Erfinder: STROEFER, Eckhard, 68163 Mannheim (DE); SCHELLING, Heiner, 67281 Kirchheim (DE); REINHARDT, Robert, 67149 Meckenheim (DE); ZOELLINGER, Michael, 73054 Eislingen (DE); BREUNINGER, Daniel, 67240 Bobenheim-Roxheim (DE); KRAEMER, Markus, 01471 Radeburg (DE); THIELE, Kai, B-2040 Antwerpen 4 (BE); VAN DEN ABEEL, Peter, B-2930 Brasschaat (BE)
(86) Internationale Anmeldenummer: PCT/EP2010/055148
(87) Internationale Veröffentlichungsnummer: WO 2010/121997

(56) Entgegenhaltungen:
- EP-A1- 1 270 544
- EP-A1- 1 375 561
- EP-A2- 0 962 448
- WO-A1-01/00569
- WO-A1-2004/014845

## Beschreibung

Die Erfindung betrifft ein Verfahren zur Herstellung von Methylendiphenyldiamin (MDA) durch Umsetzung von Formaldehyd und Anilin in Gegenwart eines sauren Katalysators, wobei der Sauerstoffgehalt im Verfahren möglichst gering gehalten wird. Weiterhin betrifft die vorliegende Erfindung ein Verfahren zur Herstellung von Methylendiphenyldiisocyanat (MDI) durch Phosgenierung von MDA. Vorzugsweise wird auch bei der MDI-Herstellung der Sauerstoffgehalt möglichst gering gehalten.

MDA ist ein Vertreter der Polyamine der Diphenylmethan-Reihe. MDA dient insbesondere als Zwischenprodukt, aus dem durch Phosgenierung das entsprechende Polyisocyanat (MDI) synthetisiert wird. MDI wiederum ist ein Ausgangsmaterial bei der Herstellung von Polyurethanen (PUR), insbesondere von Polyurethanschäumen. Ein generelles Problem von sowohl MDA als auch MDI ist die (mangelnde) Farbgüte. Je nach verwendetem Herstellungsprozess enthalten MDA bzw. MDI diverse Nebenprodukte, die das entsprechende Produkt bzw. die daraus hergestellten Polyurethane verfärben. Folglich ist bei der Herstellung von MDA bzw. MDI darauf zu achten, dass möglichst farbgute Ware hergestellt wird. Verfahren zur Herstellung von MDA bzw. von MDI sind seit längerem bekannt.

So betrifft EP-A 1 270 544 ein Verfahren zur Herstellung von Polyisocyanaten wie MDI mit einem Gehalt an hydrolisierbarem Chlor von < 0,1 % und einer lodfarbzahl in einer Verdünnung von 1 : 5 in Monochlorbenzol von < 30, erhältlich durch Phosgenierung von MDA. MDA wiederum wird hergestellt durch Umsetzung von Anilin mit Formaldehyd in Gegenwart saurer Katalysatoren, wobei in einem halbkontinuierlichen Verfahren Anilin und gegebenenfalls saurer Katalysator vorgelegt, Formaldehyd und gegebenenfalls saurer Katalysator durch ein Mischorgan in einem Kreislauf, in dem Anilin, gegebenenfalls saurer Katalysator, gegebenenfalls bereits zugegebenes Formaldehyd kreisläufig bewegt werden, eingespeist und nach Einspeisung von mindestens 50 % der gesamt einzuspeisenden Formaldehydmenge die Reaktionsmischung auf eine Temperatur von > 75°C temperiert werden.

EP-A 0 451 442 betrifft ein Verfahren zur kontinuierlichen Herstellung von Polyaminen der Diphenylmethan-Reihe, wobei N-Methylmethylendianilin in Konzentrationen von nicht größer als 0,18 % enthalten ist. Die Polyamine werden hergestellt, indem Anilin und Formaldehyd in Gegenwart von Salzsäure in mehreren Schritten und unter bestimmten molaren Beziehungen in speziell festgelegten Temperaturintervallen miteinander vermischt werden.

Die Herstellung von hellen - also farbstabilen - Isocyanaten wie MDI wird in WO 01/00569 offenbart, wobei bei der Phosgenierung Phosgen verwendet wird, das nur eine Höchstmenge an Brom und/oder Iod enthalten darf. Ein alternatives Verfahren zur Herstellung von hellen Isocyanaten wird in WO 2004/014845 beschrieben, wobei Phosgen aus 2 Teilmengen Chlor hergestellt wird. Der bei der Phosgenierung frei werdende Chlorwasserstoff wird durch katalytische Oxidation mit Sauerstoff zu Chlor umgesetzt, das wiederum in das Verfahren rückgeführt wird.

EP-A 1 288 190 betrifft ein Verfahren zur Herstellung von Polyaminen der Diphenylmethan-Reihe sowie die weiterführende Umsetzung mit Phosgen zu Polyisocyanaten der Diphenylmethan-Reihe mit einem verminderten Farbwert. In diesem Verfahren wird nach der Umsetzung von Anilin und Formaldehyd in Gegenwart eines sauren Katalysators das Reaktionsgemisch mit einer Base bei einer Temperatur von oberhalb 110°C neutralisiert oder das Reaktionsgemisch nach der Neutralisation mit einer Base auf eine Temperatur von oberhalb 110°C aufgeheizt. Ein sinngemäßes Verfahren wird in EP-A 1 344 766 offenbart, worin nach der Neutralisation mit der Base in eine wässrige und eine organische Phase getrennt und die organische Phase wiederum mit einer Base versetzt wird. Gemäß dem in US-A 2004/0002579 beschriebenen Verfahren ist bei der Neutralisation mit der Base zusätzlich mindestens ein Alkohol vorhanden.

Gemäß DE-A 42 08 359 werden farbstabile Isocyanate wie MDI durch Wasserstoffbehandlung des entsprechenden Amins bei einem Druck von 3 bis 150 bar, einer Temperatur von 100 bis 180°C unter Verwendung von Katalysatoren für 15 bis 4 h hergestellt. Im Verfahren gemäß DE 198 15 055 wird die Farbaufhellung von polymeren MDI durch Bestrahlung mit Licht einer Wellenlänge von 250 bis 2500 nm erzielt. Gemäß US-A 5,312,971 wird die Phosgenierung bei der MDI-Herstellung in Gegenwart von Reduktionsmitteln durchgeführt.

EP-A 0 445 602 offenbart Verfahren zur Herstellung von MDI mit einer verminderten lodfarbzahl, wobei der Reaktionsmischung nach beendeter Phosgenierung niedermolekulare Alkanole und/oder mehrwertige Alkohole in wirksamer Menge zugesetzt werden. Ein sinngemäßes Verfahren ist in DE-A 42 32 769 offenbart, wonach der Reaktionsmischung nach beendeter Phosgenierung Amine, Harnstoffverbindungen oder Mischungen davon zugesetzt werden.

Wie vorstehend ausgeführt, werden im Stand der Technik die unterschiedlichsten Maßnahmen getroffen, um farbstabiles MDA bzw. MDI herzustellen. Nirgendwo ist jedoch offenbart, dass die Gegenwart von Sauerstoff bei der MDA- bzw. MDI-Herstellung einen Einfluss auf die Farbstabilität dieser beiden Verbindungen hat.

Die der vorliegenden Erfindung zugrunde liegende Aufgabe besteht in der Bereitstellung eines wirtschaftlichen Verfahrens zur Herstellung von MDA bzw. MDI.

Die Aufgabe wird gelöst durch ein Verfahren zur Herstellung von Methylendiphenyldiamin (MDA) durch Umsetzung von Formaldehyd und Anilin in Gegenwart eines sauren Katalysators, dadurch gekenntzeichnet, dass bei der Herstellung von MDA der Sauerstoffgehalt im Verfahren ≤ 500 ppm, bezogen auf alle sich im Verfahren befindlichen Verbindungen, beträgt. Aus MDA kann wiederum durch Phosgenierung Methylendiphenyldiisocyanat (MDI) hergestellt werden.

Das erfindungsgemäße Verfahren hat den Vorteil, dass auf einfachem Wege die Farbstabilität von MDA beziehungsweise MDI verbessert werden kann. Vorteilhafterweise wird bereits auf der MDA-Stufe die Bildung von Nebenprodukten unterbunden beziehungsweise vermindert. So enthält das mit dem erfindungsgemäßen Verfahren hergestellte MDA bzw. MDI einen geringen Anteil an farbgebenden Substanzen wie Diarylmethan-Farbstoffe (beispielsweise "Michlers Hydrolblau") oder Triarylmethan-Farbstoffe (beispielsweise "Kristallviolett"). Durch das erfindungsgemäße Verfahren können farbstabiles MDA bzw. MDI hergestellt werden, ohne dass dabei Fremdsubstanzen zugegeben werden müssen, die zwar einen positiven Effekt auf die Farbgüte, aber einen negativen Effekt auf die Reinheit des entsprechenden Produktes haben.

Nachfolgend wird das erfindungsgemäße Verfahren zur Herstellung von MDA beziehungsweise MDI näher beschrieben.

Verfahren zur Herstellung der Edukte Anilin und Formaldehyd sind dem Fachmann bekannt. Prinzipiell können Anilin oder Formaldehyd nach beliebigen Verfahren hergestellt werden. Vorteilhafterweise wird Anilin durch katalytische Hydrierung von Nitrobenzol in der Gasphase im Festbett oder Wirbelbett hergestellt. Dafür geeignete Katalysatoren sind beispielsweise in US-A 3,538,018 beschrieben. Formaldehyd wird vorzugsweise als Lösung, beispielsweise wässrige Lösung, eingesetzt. Die Formaldehyd-lösung wird vorteilhaft hergestellt nach dem Silberkontaktverfahren mit Wasserstoffüberschuss (reduzierende Bedingungen) im Reaktionsaustrag der Formaldehydsynthese. Reduzierende Bedingungen sind besser geeignet als oxidierende Bedingungen nach dem Formox-Verfahren mit Sauerstoffüberschuss im Reaktionsaustrag der Formaldehydsynthese.

Als saure Katalysatoren können starke organische oder starke anorganische Säuren eingesetzt werden. Geeignete Säuren sind beispielsweise Salzsäure (HCl), Schwefelsäure, Phosphorsäure oder Methansulfonsäure. Bevorzugt wird im erfindungsgemäßen Verfahren wässrige Salzsäure eingesetzt, üblicherweise in Konzentrationen von 25 bis 36 Gew.-%. Gegebenenfalls kann auch gasförmige HCl eingesetzt werden. Vorzugsweise wird im erfindungsgemäßen Verfahren der saure Katalysator in wässriger Form eingesetzt.

Im erfindungsgemäßen Verfahren beträgt der Sauerstoffgehalt bei der Herstellung von MDA ≤ 500 ppm. Der vorgenannte Wert der Obergrenze des Sauerstoffgehaltes bei der Herstellung von MDA bezieht sich auf alle sich im Verfahren befindlichen Verbindungen. Sich im Verfahren befindliche Verbindungen sind beispielsweise die Edukte Formaldehyd und Anilin, der saure Katalysator, etwaiges Lösungsmittel, das Produkt MDA, etwaige Nebenprodukte oder sonstige im Verfahren präsente Stoffe wie Additive oder Schutzgase.

In anderen Worten ausgedrückt bedeutet dies, dass im erfindungsgemäßen Verfahren die Herstellung von MDA nahezu oder vollständig sauerstofffrei durchgeführt wird. Dies kann beispielsweise erreicht werden durch Minimieren des Eindringens von Leckluft in das Verfahren und unter Befreiung eines, mehrerer oder aller Stoffströme, die dem Verfahren bzw. der Reaktion als solcher zugeführt werden, von Sauerstoff. Dies betrifft insbesondere die Leckgasströme an Vakuumkolonnen und/oder das Befreien von Feedströmen von darin eingelöstem Sauerstoff. Auf diese Weise kann die Farbkörperbildung im Produkt deutlich reduziert werden.

Die vorgenannten Sauerstoffhöchstwerte des erfindungsgemäßen Verfahrens werden somit vorzugsweise in dem Reaktionsgefäß nicht überschritten, in dem die MDA-Herstellung durchgeführt wird, beispielsweise in einem Reaktor. Weiterhin ist es bevorzugt, die Sauerstoffhöchstwerte in einem oder mehreren Stoffströmen, insbesondere der Feedströme, nicht zu überschreiten. Dies gilt auch für sämtliche Apparaturen, durch die das erhaltene Produkt (MDA) aufgereinigt, geleitet oder gelagert wird, beispielsweise Vakuumkolonnen, sonstige Kolonnen, Vorratsgefäße, Zuleitungen oder Rückleitungen. In vorteilhafter Weise kann im erfindungsgemäßen Verfahren der Sauerstoffgehalt durch Entgasen auf einen Sauerstoffhöchstwert von (beispielsweise) ≤ 500 ppm reduziert werden. Wie vorstehend bereits ausgeführt, kann ein erhöhter Sauerstoffgehalt von der Leckluft herrühren oder in gelöster Form bereits in den chemischen Verbindungen vorliegen, die im Verfahren eingesetzt werden. Das Entgasen von beispielsweise eingelöstem Sauerstoff kann mit allen dem Fachmann bekannten Methoden durchgeführt werden, beispielsweise durch Strippen, Destillieren, Absorbieren oder ein Membranverfahren. Vorzugsweise erfolgt das Entgasen durch Strippen. Vorzugsweise wird dies unter Verwendung von einer oder mehreren Strippkolonnen durchgeführt. Die Strippkolonne kann als Füllkörperkolonne vorliegen, als Strippgas ist Stickstoff geeignet. Weiterhin kann das Verfahren unter einer Inertgasatmosphäre durchgeführt (beziehungsweise gehalten) werden.

Der Sauerstoffgehalt kann beispielsweise im eingesetzten Anilin-, Formaldehyd oder saureren Katalysator-Feedstrom (Einspeisungsstrom) durch Entgasen reduziert werden. Ebenso kann der Sauerstoffgehalt auch durch Entgasen von rückgeführten Strömen, beispielsweise von nicht umgesetztem Anilin durch Entgasen reduziert werden. In einer Ausführungsform der vorliegenden Erfindung wird der Sauerstoffgehalt bei der Rückführung von nicht umgesetztem Anilin, vorzugsweise durch Entgasen, insbesondere durch Strippen, reduziert. In einer weiteren Ausführungsform wird der Sauerstoffgehalt im sauren Katalysator, der in wässriger Form vorliegt, reduziert, vorzugsweise durch Entgasen, insbesondere durch Strippen. Vorzugsweise handelt es sich bei dem sauren Katalysator in wässriger Form um Salzsäure.

In einer Ausführungsform wird Formaldehyd als Formaldehyd-Lösung in sauerstoffarmer reduzierter Form bereitgestellt. Weiterhin kann Anilin in sauerstoffarmer reduzierter Form bereitgestellt werden. Vorzugsweise erfolgt die Lagerung und Bereithaltung der Edukte beziehungsweise des Produktes unter Inertgas, beispielsweise Stickstoff oder Argon, insbesondere Stickstoff.

Das erfindungsgemäße Verfahren kann so durchgeführt werden, dass mindestens eine der im Verfahren eingesetzten Vorrichtungen, insbesondere das Reaktionsgefäß beziehungsweise der Reaktor, bei Überdruck betrieben wird. Weiterhin kann mindestens eine der im Verfahren eingesetzten Vorrichtungen mit einem Inertgasmantel versehen sein, beispielsweise eine Ummantelung von Vakuumkolonnen mit Inertgas (Doppelmantelkolonnen). Diese Ummantelung kann ganz erfolgen oder partiell an besonders kritischen Stellen wie zum Beispiel Flanschen.

Die Herstellung von MDA erfolgt im erfindungsgemäßen Verfahren im Übrigen (beispielsweise hinsichtlich Druck, Temperatur, Vorrichtungen, Aufreinigungen oder etwaige Zusatzstoffe/Lösungsmittel) nach dem Fachmann bekannten Methoden. Im erfindungsgemäßen Verfahren wird 4,4'-MDA als Hauptprodukt sowie 2,4'-MDA und 2,2'-MDA als Nebenprodukte hergestellt. Beispielsweise kann das erfindungsgemäße Verfahren auch über die Zwischenstufe eines Aminals durchgeführt werden, indem Formaldehyd und Anilin zunächst direkt umgesetzt werden und die Zugabe des sauren Katalysators erst nach Ausbildung des Aminals erfolgt. Aus dem Aminal wird wiederum durch zumindest zweifache Umlagerung MDA gebildet. Solche Verfahren zur Herstellung von MDA über die Aminal-Zwischenstufe sind dem Fachmann bekannt. Weiterhin kann nach der Ausbildung von MDA dem Reaktionsgemisch eine Base, beispielsweise wässrige NaOH, zugegeben werden, wodurch eine Neutralisation bzw. Teilneutralisation des Reaktionsgemisches erfolgt. Die Basenzugabe kann bei einer Temperatur von oberhalb 110°C durchgeführt werden, alternativ kann die Temperaturerhöhung auf einen Wert von oberhalb 110°C erst nach der Basenzugabe erfolgen. Weiterhin kann das Reaktionsgemisch nach der Basenzugabe in eine wässrige und eine organische Phase getrennt werden und die organische Phase erneut mit einer Base versetzt werden.

In einer Ausführungsform der vorliegenden Erfindung wird bei der MDA-Herstellung in einem halbkontinuierlichen Verfahren Anilin und gegebenenfalls saurer Katalysator vorgelegt, Formaldehyd und gegebenenfalls saurer Katalysator durch ein Mischorgan in einen Kreislauf, in dem Anilin, gegebenenfalls saurer Katalysator und gegebenenfalls bereits zugegebenes Formaldehyd kreisläufig bewegt werden, eingespeist und nach Einspeisung von mindestens 50 % der gesamt einzuspeisenden Formaldehydmenge die Reaktionsmischung auf eine Temperatur von > 75°C temperiert wird.

In einer weiteren Ausführungsform der vorliegenden Erfindung wird die MDA-Herstellung in Gegenwart von Salzsäure (als saurem Katalysator) gemäß den nachfolgenden Ziffern a) bis d) durchgeführt.
a) die Menge an Salzsäure ist im Bereich von 0,05 bis 0,5 Mol pro Mol Anilin,
b) die Menge an Anilin ist im Bereich von 1,5 bis 4 Mol pro Mol Formaldehyd,
c) die MDA-Herstellung ist in mindestens vier Stufen unterteilt, wobei die erste Stufe bei 20 bis 50°C und einem Wasser/Anilin-Verhältnis im Bereich von 1,3 bis 2,5 Mol, die zweite Stufe bei 40 bis 70°C und einem Wasser/Anilin-Verhältnis im Bereich von 1,9 bis 5 Mol, die dritte Stufe bei 50 bis 90°C und einem Wasser/Anilin-Verhältnis im Bereich von 2,4 bis 5,7 Mol und die vierte Stufe bei einer Temperatur von mindestens 110°C durchgeführt werden und
d) Formaldehyd in mindestens drei Fraktionen in den Stufen gemäß Ziffer c) eingesetzt wird.

In einer bevorzugten Ausführungsform der vorliegenden Erfindung wird das MDA durch Phosgenierung zu Methylendiphenyldiisocyanat (MDI) umgesetzt. Verfahren zur Herstellung von MDI aus MDA durch Phosgenierung sind dem Fachmann bekannt. Vorzugsweise wird das erfindungsgemäße Verfahren in dieser Ausführungsform so durchgeführt, dass bei der Herstellung von MDI aus MDA der Sauerstoffgehalt im Verfahren (für den MDI-Teilschritt) < 10 000 ppm, bezogen auf alle sich im Verfahren befindlichen Verbindungen, beträgt. Vorzugsweise beträgt der Sauerstoffgehalt in diesem Teilschritt ≤ 1000 ppm, insbesondere ≤ 500 ppm. Weiterhin ist es bevorzugt, dass der Sauerstoffgehalt in beiden Teilschritten des erfindungsgemäßen Verfahrens (MDA-Herstellung und MDI-Herstellung) ≤ 500 ppm, bezogen auf alle sich im Verfahren befindlichen Verbindungen, beträgt.

Die MDA-Herstellung und die MDI-Herstellung können räumlich und/oder zeitlich getrennt voneinander durchgeführt werden. Denkbar ist aber auch ein kontinuierliches Verfahren, in dem frisch hergestelltes MDA direkt zu MDI weiter umgesetzt wird. Dies kann wiederum im selben Reaktionsgefäß (Vorrichtung) durchgeführt werden, vorzugsweise erfolgt die MDA-Herstellung und die MDI-Herstellung jedoch in unterschiedlichen Vorrichtungen (Apparaturen), die beispielsweise durch Leitungen miteinander verbunden sind. Die Reduktion des Sauerstoffgehaltes bei der MDI-Herstellung wird im erfindungsgemäßen Verfahren entsprechend der Reduktion des Sauerstoffgehaltes bei der MDA-Herstellung durchgeführt. Beispielsweise kann der Sauerstoffgehalt durch Entgasen, insbesondere durch Strippen bei der MDI-Herstellung reduziert werden, also ein Sauerstoffgehalt von beispielsweise < 10 000 ppm eingestellt werden.

Im Übrigen kann die Phosgenierung (Umsetzung von MDA mit Phosgen zu MDI) nach allen dem Fachmann bekannten Methoden durchgeführt werden, beispielsweise hinsichtlich Druck, Temperatur, Lösungsmittel, Apparatur, Aufreinigung etc.. Entsprechende Parameter sind beispielsweise in EP-A 1 270 544 beschrieben.

Im erfindungsgemäßen Verfahren kann die Phosgenierung beispielsweise unter Verwendung eines üblichen, bevorzugt inerten Lösungsmittels durchgeführt werden. Geeignete Lösungsmittel sind beispielsweise Monochlorbenzol (MCB), Dichlorbenzol oder sonstige chlorierte, aromatische Kohlenwasserstoffe wie Toluol oder Xylol. Bei der Phosgenierung werden vorzugsweise Temperaturen von 70 bis 120°C und Drücke von 8 bis 5 bar eingestellt. Die Phosgenierung kann in einer oder mehreren Stufen durchgeführt werden. Beispielsweise kann die Phosgenierung durch eine zweistufige Umsetzung in Gegenwart mindestens eines inerten organischen Lösungsmittels durchgeführt werden, wobei die erste Stufe der Phosgenierung in einem statischen Mischer und die zweite Stufe der Phosgenierung in einem Verweilzeitapparat erfolgt.

Entsprechend dem eingesetzten MDA werden im erfindungsgemäßen Verfahren durch die Phosgenierung die entsprechenden MDI-Isomere 2,2'-, 2,4'- und/oder 4,4'-MDI hergestellt.

In einer Ausführungsform der vorliegenden Erfindung enthält das bei der Phosgenierung eingesetzten Phosgen weniger als 50 ppm Brom oder Iod oder deren Gemisch. Brom oder Iod können dabei in molekularer oder gebundener Form vorliegen.

In einer weiteren Ausführungsform der vorliegenden Erfindung umfasst die Phosgenierung die nachfolgenden Schritte a) bis h):
(a) Bereitstellung einer ersten Teilmenge Chlor, wobei das Chlor der ersten Teilmenge einen Gehalt an freiem oder gebundenem Brom und Iod von < 400 ppm aufweist;
(b) Bereitstellung einer zweiten Teilmenge Chlor;
(c) Umsetzung der ersten und der zweiten Teilmenge Chlor mit Kohlenmonoxid zu Phosgen;
(d) Umsetzung des Phosgens aus Schritt (c) mit MDA zu MDI und Chlorwasserstoff;
(e) Abtrennung und gegebenenfalls Reinigung des in Schritt (d) gebildeten MDI;
(f) Abtrennung und gegebenenfalls Reinigung des in Schritt (d) gebildeten Chlorwasserstoffs;
(g) katalytische Oxidation zumindest eines Teils des in Schritt (e) abgetrennten Chlorwasserstoffs mit Sauerstoff zu Chlor;
(h) Abtrennung des in Schritt (g) gebildeten Chlors und Einsatz zumindest einer Teilmenge des abgetrennten Chlors als zweite Teilmenge Chlor in Schritt (b).

Vorzugsweise wird dabei das Chlor der ersten Teilmenge durch Elektrolyse einer Chloridionen enthaltenen Lösung erhalten. Weiterhin ist es bevorzugt, dass das Chlor der ersten Teilmenge in einer Abreicherungsstufe an Brom oder Iod abgereichert wird. Vorzugsweise wird die Chlorwasserstoff-Oxidation heterogenkatalytisch durchgeführt. Für die Chlorwasserstoff-Oxidation eignen sich Katalysatoren enthaltend Rutheniumoxid auf einem Träger ausgewählt aus der Gruppe bestehend aus Siliciumdioxid, Aluminiumoxid, Titandioxid, Zirkondioxid und deren Gemische. Vorzugsweise wird die Chlorwasserstoffoxidation in einem Festbett- oder Wirbelbettreaktor durchgeführt.

In einer weiteren Ausführungsform der vorliegenden Erfindung werden der Reaktionsmischung nach beendeter Phosgenierung niedermolekulare Alkanole und/oder mehrwertige Alkohole zugesetzt. Dabei kann gegebenenfalls auch überschüssiges Phosgen sowie das Lösungsmittel abgetrennt werden und/oder eine thermische Behandlung des Reaktionsproduktes erfolgen. Niedermolekulare Alkanole sind sekundäre, tertiäre und vorzugsweise primäre Alkanole mit verzweigten oder vorzugsweise linearen Alkylresten mit 1 bis 10 Kohlenstoffatomen. Beispielhaft sind hierfür Methanol, Ethanol, n- und iso-Propanol, n-Butanol etc.. Geeignete mehrwertige Alkohole sind zweckmäßigerweise 2- bis 8-wertig und weisen ein Molekulargewicht von 60 bis 350 auf. Beispielhaft genannt sind 1,4-Butandiol, Triethanolpropan, Glycerine oder Pentaerythrit.

Gegebenenfalls kann nach Abtrennung des überschüssigen Phosgens und des inerten organischen Lösungsmittels und vor der thermischen Behandlung des Reaktionsproduktes mindestens ein Antioxidans auf Phenolbasis und/oder mindestens ein Arylphosphit zugegeben werden. Vorzugsweise ist das Antioxidans auf Phenolbasis Di-tert.-butyl-p-Kresol und das Arylphosphit ist Triphenylphosphit. Gegebenenfalls können nach beendeter Phosgenierung auch Amine, Harnstoffverbindungen oder Mischungen davon zugegeben werden.

Die Erfindung wird anhand der nachfolgenden Beispiele verdeutlicht.

### Beispiel 1

In einen Kolben mit Gasein- und Gasausleitung sowie KPG-Rührer werden unter Stickstoffatmosphäre 70 g eines frisch hergestellten polymeren Methylendiphenylendiamin (MDA) eingewogen und unter Rühren auf 50°C temperiert. Anschließend lässt man durch die Gaseinleitung trockenen sauerstoffhaltigen Stickstoff (siehe Tabelle 1) unter stets gleichen Gasvolumenströmen einströmen. Nach genau 60 min wird die Gaseinleitung abgebrochen und mit reinem trockenem Stickstoff gespült. Anschließend wird mit 1300 mL trockenem entgastem Monochlorbenzol (MCB) verdünnt und die Lösung in einen Tropftrichter überführt.

In einen Reaktor werden 1300 mL MCB vorgelegt und bei Raumtemperatur 160 g Phosgen einkondensiert. Anschließend wird aus dem Tropftrichter die Lösung aus 1300 mL MCB und 70 g MDA bei 50°C innerhalb von 60 min unter Rühren in die Phosgenlösung getropft. Danach wird auf 110°C erwärmt bis die Suspension klar geworden ist. Anschließend wird das Lösungsmittel unter vermindertem Druck bis zu einer Sumpftemperatur von 100°C abdestilliert. Anschließend wird das restliche Lösungsmittel bei 5 mbar und mindestens 60°C vollständig entfernt. Das so erhaltene Isocyanat wird in einen Kolben überführt und mit Hilfe eines Rotationsverdampfers 45 min bei 100 °C und einem Vakuum von 5 mbar behandelt. Danach wird bei gleichem Vakuum 60 min bis zum ersten Produktübergang erhitzt. Nach Abkühlen wird 1 g des erhaltenen Roh-MDIs in 5 g MCB gelöst und mit einem Dr. Lange (LICO 500) Spektralphotometer untersucht. In der folgenden Tabelle sind die erhaltenen Werte für die Jodfarbzahl (JFZ), der L*, a* und der b*-Wert eingetragen.

**Tabelle 1**

| | CIE-LAB Farben nach 60 min bei 180 °C | | | |
|---|---|---|---|---|
| O2 - Konz. in Stickstoff | JFZ | L* | a* | b* |
| 0 ppm | 28,0 | 74,8 | 3,4 | 58,8 |
| 50 ppm | 29,9 | 70,9 | 4,7 | 61,4 |
| 100 ppm | 32,4 | 67,2 | 6,8 | 63,8 |
| 1000 ppm (Vergleich) | 38,6 | 61,2 | 10,3 | 68,5 |
| 5000 ppm (Vergleich) | 44,9 | 55,8 | 11,5 | 71,5 |
| 1% (Vergleich) | 49,6 | 50,6 | 12,4 | 73,1 |
| 21% (Vergleich) | 61,0 | 41,0 | 17,2 | 81,3 |

Nach dem CIE-LAB-Farbsystem sind folgende Werte für polymeres MDI besonders vorteilhaft:
Der L* - Wert sollte im Idealfall ≤ 100 sein, der a* - Wert sollte im Idealfall im Bereich von -10 bis 0 liegen, der b* - Wert sollte nicht größer als 65 sein und die Jodfarbzahl sollte möglichst klein sein.

Aus den vorliegenden Daten kann man klar den Trend erkennen, dass es vorteilhaft ist, wenn MDA möglichst wenig Sauerstoff ausgesetzt ist. Aus Tabelle 1 ist deutlich zu erkennen, dass die größte Änderung der einzelnen Farbwerte vor allem bei den niedrigen Konzentrationen von eingetragenem Sauerstoff auftreten.

Somit ist klar erwiesen, dass es vorteilhaft ist, wenn der Sauerstoffgehalt im Reaktionsgefäß bzw. an jeder Stelle des Prozesses ≤ 500 ppm beträgt, um möglichst gute Farbwerte im Sinne des CIE-LAB-Farbsystems zu bekommen.

## Patentansprüche

1. Verfahren zur Herstellung von Methylendiphenyldiamin (MDA) durch Umsetzung von Formaldehyd und Anilin in Gegenwart eines sauren Katalysators, **dadurch gekennzeichnet, dass** bei der Herstellung von MDA der Sauerstoffgehalt im Verfahren s 500 ppm, bezogen auf alle sich im Verfahren befindlichen Verbindungen, beträgt

2. Verfahren gemäß Anspruch 1, **dadurch gekennzeichnet, dass** MDA durch Phosgenierung zu Methylendiphenyldiisocyanat (MDI) umgesetzt wird.

3. Verfahren gemäß Anspruch 2, **dadurch gekennzeichnet, dass** bei der Herstellung von MDI der Sauerstoffgehalt im Verfahren ≤ 500 ppm, bezogen auf alle sich im Verfahren befindlichen Verbindungen, beträgt.

4. Verfahren gemäß einem der Ansprüche 1 bis 3, **dadurch gekennzeichnet, dass** der Sauerstoffgehalt im Verfahren durch Entgasen, insbesondere durch Strippen, reduziert wird und/oder das Verfahren unter einer Inertgasatmosphäre durchgeführt wird.

5. Verfahren gemäß einem der Ansprüche 1 bis 4, **dadurch gekennzeichnet, dass** der Sauerstoffgehalt bei der Rückführung von nicht umgesetztem Anilin und/oder im sauren Katalysator, der in wässriger Form vorliegt, reduziert wird.

6. Verfahren gemäß einem der Ansprüche 1 bis 5, **dadurch gekennzeichnet, dass** mindestens eine der im Verfahren eingesetzten Vorrichtungen mit einem Inertgasmantel versehen ist und/oder bei Überdruck betrieben wird.

7. Verfahren gemäß einem der Ansprüche 2 bis 6, **dadurch gekennzeichnet, dass** das bei der Phosgenierung eingesetzte Phosgen weniger als 50 ppm Brom oder Iod oder deren Gemisch in molekularer oder gebundener Form enthält.

8. Verfahren gemäß einem der Ansprüche 2 bis 6, **dadurch gekennzeichnet, dass** die Phosgenierung die nachfolgenden Schritte a) bis h) umfasst:
(a) Bereitstellung einer ersten Teilmenge Chlor, wobei das Chlor der ersten Teilmenge einen Gehalt an freiem oder gebundenem Brom und Iod von < 400 ppm aufweist;
(b) Bereitstellung einer zweiten Teilmenge Chlor;
(c) Umsetzung der ersten und der zweiten Teilmenge Chlor mit Kohlenmonoxid zu Phosgen;
(d) Umsetzung des Phosgens aus Schritt (c) mit MDA zu MDI und Chlorwasserstoff;
(e) Abtrennung und gegebenenfalls Reinigung des in Schritt (d) gebildeten MDI;
(f) Abtrennung und gegebenenfalls Reinigung des in Schritt (d) gebildeten Chlorwasserstoffs;
(g) katalytische Oxidation zumindest eines Teils des in Schritt (e) abgetrennten Chlorwasserstoffs mit Sauerstoff zu Chlor;
(h) Abtrennung des in Schritt (g) gebildeten Chlors und Einsatz zumindest einer Teilmenge des abgetrennten Chlors als zweite Teilmenge Chlor in Schritt (b).

9. Verfahren gemäß einem der Ansprüche 1 bis 8, **dadurch gekennzeichnet, dass** bei der MDA-Herstellung in einem halbkontinuierlichen Verfahren Anilin und gegebenenfalls saurer Katalysator vorgelegt, Formaldehyd und gegebenenfalls saurer Katalysator durch ein Mischorgan in einen Kreislauf, in dem Anilin, gegebenenfalls saurer Katalysator und gegebenenfalls bereits zugegebenes Formaldehyd kreisläufig bewegt werden, eingespeist und nach Einspeisung von mindestens 50 % der gesamt einzuspeisenden Formaldehydmenge die Reaktionsmischung auf eine Temperatur von > 75°C temperiert wird.

10. Verfahren gemäß einem der Ansprüche 1 bis 8, **dadurch gekennzeichnet, dass** die MDA-Herstellung in Gegenwart von Salzsäure als sauren Katalysator gemäß den nachfolgenden Ziffern a) bis d) durchgeführt wird:
a) die Menge an Salzsäure ist im Bereich von 0,05 bis 0,5 Mol pro Mol Anilin,
b) die Menge an Anilin ist im Bereich von 1,5 bis 4 Mol pro Mol Formaldehyd,
c) die MDA-Herstellung ist in mindestens vier Stufen unterteilt, wobei die erste Stufe bei 20 bis 50°C und einem Wasser/Anilin-Verhältnis im Bereich von 1,3 bis 2,5 Mol, die zweite Stufe bei 40 bis 70°C und einem Wasser/Anilin-Verhältnis im Bereich von 1,9 bis 5 Mol, die dritte Stufe bei 50 bis 90°C und einem Wasser/Anilin-Verhältnis im Bereich von 2,4 bis 5,7 Mol und die vierte Stufe bei einer Temperatur von mindestens 110°C durchgeführt werden und
d) Formaldehyd in mindestens drei Fraktionen in den Stufen gemäß Ziffer c) eingesetzt wird.

11. Verfahren gemäß einem der Ansprüche 2 bis 10, **dadurch gekennzeichnet, dass** der Reaktionsmischung nach beendeter Phosgenierung niedermolekulare Alkanole und/oder mehrwertige Alkohole zugesetzt werden,
wobei niedermolekulare Alkanole sekundäre, tertiäre und primäre Alkanole mit verzweigten oder linearen Alkylresten mit 1 bis 10 Kohlenstoffatomen sind.

## Claims

1. A process for preparing methylenediphenyldiamine (MDA) by reacting formaldehyde and aniline in the presence of an acidic catalyst, wherein the oxygen content in the process for preparing MDA is ≤ 500 ppm, based on all compounds present in the process.

2. The process according to claim 1, wherein MDA is converted by phosgenation to methylenediphenyl diisocyanate (MDI).

3. The process according to claim 2, wherein the oxygen content in the process for preparing MDI is ≤ 500 ppm, based on all compounds present in the process.

4. The process according to any one of claims 1 to 3, wherein the oxygen content is reduced in the process by degassing, especially by stripping, and/or the process is performed under an inert gas atmosphere.

5. The process according to any one of claims 1 to 4, wherein the oxygen content is reduced in the course of recycling of unconverted aniline and/or in the acidic catalyst which is present in aqueous form.

6. The process according to any one of claims 1 to 5, wherein at least one of the apparatuses used in the process is provided with an inert gas jacket and/or is operated at elevated pressure.

7. The process according to any one of claims 2 to 6, wherein the phosgene used in the phosgenation comprises less than 50 ppm of bromine or iodine or a mixture thereof in molecular or bound form.

8. The process according to any one of claims 2 to 6, wherein the phosgenation comprises the following steps a) to h):
(a) providing a first portion of chlorine, the chlorine of the first portion having a content of free or bound bromine and iodine of < 400 ppm;
(b) providing a second portion of chlorine;
(c) reacting the first and second portions of chlorine with carbon monoxide to give phosgene;
(d) reacting the phosgene from step (c) with MDA to give MDI and hydrogen chloride;
(e) removing and optionally purifying the MDI formed in step (d);
(f) removing and optionally purifying the hydrogen chloride formed in step (d);
(g) catalytically oxidizing at least a portion of the hydrogen chloride removed in step (e) with oxygen to give chlorine;
(h) removing the chlorine formed in step (g) and using at least a portion of the chlorine removed as the second portion of chlorine in step (b).

9. The process according to any one of claims 1 to 8, wherein MDA preparation in a semicontinuous process comprises initially charging aniline and optionally acidic catalyst, feeding formaldehyde and optionally acidic catalyst through a mixing unit into a circulation system in which aniline, optionally acidic catalyst and optionally formaldehyde which has already been added are circulated, and, after feeding in at least 50% of the total amount of formaldehyde to be fed in, adjusting the reaction mixture to a temperature of > 75°C.

10. The process according to any one of claims 1 to 8, wherein the MDA preparation is performed in the presence of hydrochloric acid as the acidic catalyst according to the following points a) to d) :
a) the amount of hydrochloric acid is in the range from 0.05 to 0.5 mol per mole of aniline,
b) the amount of aniline is in the range from 1.5 to 4 mol per mole of formaldehyde,
c) the MDA preparation is divided into at least four stages, the first stage being performed at 20 to 50°C and a water/aniline ratio in the range from 1.3 to 2.5 mol, the second stage at 40 to 70°C and a water/aniline ratio in the range from 1.9 to 5 mol, the third stage at 50 to 90°C and a water/aniline ratio in the range from 2.4 to 5.7 mol, and the fourth stage at a temperature of at least 110°C, and
d) formaldehyde is used in at least three fractions in the stages according to point c).

11. The process according to any one of claims 2 to 10, wherein low molecular weight alkanols and/or polyhydric alcohols are added to the reaction mixture after the phosgenation has ended, low molecular weight alkanols being secondary, tertiary and primary alkanols with branched or linear alkyl radicals with 1 to 10 carbon atoms.

## Revendications

1. Procédé pour la préparation de méthylènediphényldiamine (MDA) par transformation de formaldéhyde et d'aniline en présence d'un catalyseur acide, **caractérisé en ce que** lors de la préparation de MDA, la teneur en oxygène dans le procédé est ≤ 500 ppm, par rapport à tous les composés se trouvant dans le procédé.

2. Procédé selon la revendication 1, **caractérisé en ce que** la MDA est transformée par phosgénation en diisocyanate de méthylènediphényle (MDI).

3. Procédé selon la revendication 2, **caractérisé en ce que** lors de la préparation de MDI, la teneur en oxygène dans le procédé est ≤ 500 ppm, par rapport à tous les composés se trouvant dans le procédé.

4. Procédé selon l'une quelconque des revendications 1 à 3, **caractérisé en ce que** la teneur en oxygène dans le procédé est réduite par dégazage, en particulier par rectification, et/ou le procédé est réalisé sous une atmosphère de gaz inerte.

5. Procédé selon l'une quelconque des revendications 1 à 4, **caractérisé en ce que** la teneur en oxygène est réduite lors du recyclage d'aniline non transformée et/ou dans le catalyseur acide, qui se trouve sous forme aqueuse.

6. Procédé selon l'une quelconque des revendications 1 à 5, **caractérisé en ce qu'**au moins un des dispositifs utilisés dans le procédé est doté d'un manteau de gaz inerte et/ou est exploité en surpression.

7. Procédé selon l'une quelconque des revendications 2 à 6, **caractérisé en ce que** le phosgène utilisé lors de la phosgénation contient moins de 50 ppm de brome ou d'iode ou de leur mélange sous forme moléculaire ou liée.

8. Procédé selon l'une quelconque des revendications 2 à 6, **caractérisé en ce que** la phosgénation comprend les étapes suivantes a) à h) :
(a) mise à disposition d'une première quantité partielle de chlore, le chlore de la première quantité partielle présentant une teneur en brome et en iode libres ou liés < 400 ppm ;
(b) mise à disposition d'une deuxième quantité partielle de chlore ;
(c) transformation de la première et de la deuxième quantité partielle de chlore avec du monoxyde de carbone en phosgène ;
(d) transformation du phosgène de l'étape (c) avec de la MDA en MDI et chlorure d'hydrogène ;
(e) séparation et le cas échéant purification du MDI formé dans l'étape (d) ;
(f) séparation et le cas échéant purification du chlorure d'hydrogène formé dans l'étape (d) ;
(g) oxydation catalytique d'au moins une partie du chlorure d'hydrogène séparé dans l'étape (e) avec de l'oxygène en chlore ;
(h) séparation du chlore formé dans l'étape (g) et utilisation d'au moins une quantité partielle du chlore séparé comme deuxième quantité partielle de chlore dans l'étape (b).

9. Procédé selon l'une quelconque des revendications 1 à 8, **caractérisé en ce qu'**on dispose au préalable, lors de la préparation de MDA, dans un procédé semi-continu, l'aniline et le cas échéant un catalyseur acide, on injecte le formaldéhyde et le cas échéant un catalyseur acide via un organe de mélange dans un circuit dans lequel l'aniline, le cas échéant le catalyseur acide et le cas échéant du formaldéhyde déjà ajouté sont déplacés en circulation et après injection d'au moins 50% de la quantité totale de formaldéhyde à injecter, le mélange réactionnel est équilibré thermiquement à une température > 75°C.

10. Procédé selon l'une quelconque des revendications 1 à 8, **caractérisé en ce que** la préparation de MDA est réalisée, en présence d'acide chlorhydrique comme catalyseur acide, selon les points a) à d) suivantes :
a) la quantité d'acide chlorhydrique est située dans la plage de 0,05 à 0,5 mole par mole d'aniline,
b) la quantité d'aniline est située dans la plage de 1,5 à 4 moles par mole de formaldéhyde,
c) la préparation de MDA est répartie en au moins quatre étages, le premier étage étant réalisé à 20 jusqu'à 50°C et à un rapport eau/aniline dans la plage de 1,3 à 2,5 moles, le deuxième étage étant réalisé à 40 jusqu'à 70°C et à un rapport eau/aniline dans la plage de 1,9 à 5 moles, le troisième étage étant réalisé à 50 jusqu'à 90°C et à un rapport eau/aniline dans la plage de 2,4 à 5,7 moles et le quatrième étage étant réalisé à une température d'au moins 110°C et
d) le formaldéhyde étant utilisé en au moins trois fractions dans les étages selon le point c).

11. Procédé selon l'une quelconque des revendications 2 à 10, **caractérisé en ce qu'**on ajoute au mélange réactionnel, après la phosgénation, des alcanols de bas poids moléculaire et/ou des alcools polyvalents, les alcanols de bas poids moléculaire étant des alcanols secondaires, tertiaires et primaires présentant des radicaux alkyle ramifiés ou linéaires comprenant 1 à 10 atomes de carbone.
